# EUROPÄISCHE PATENTSCHRIFT

(11) **EP 2 425 161 B1**
(45) Veröffentlichungstag und Bekanntmachung des Hinweises auf die Patenterteilung: **06.03.2013**
(21) Anmeldenummer: 10712732.6
(22) Anmeldetag: 29.03.2010
(51) Int. Cl.: F16K 7/06, A61M 39/28

(54) **VORRICHTUNG ZUR REGULIERUNG EINES VOLUMENSTROMS IN EINEM SCHLAUCH**
REGULATION DEVICE FOR CONTROLLING THE FLOW IN A TUBE
DISPOSITIF POUR REGULARISER LE DEBIT DANS UN TUYAU

(30) Priorität: 30.04.2009 DE 202009004772 U
(43) Veröffentlichungstag der Anmeldung: 07.03.2012
(73) Patentinhaber: Hopf, Hans-Jürgen, 90513 Zirndorf (DE)
(72) Erfinder: HOPF, Hans-Jürgen, 90513 Zirndorf (DE); HOPF, Michael, 90513 Zirndorf (DE); HOPF, Alexander, 90492 Nürnberg (DE)
(74) Vertreter: Wittmann, Ernst-Ulrich
(86) Internationale Anmeldenummer: PCT/EP2010/054120
(87) Internationale Veröffentlichungsnummer: WO 2010/124919

(56) Entgegenhaltungen:
- WO-A1-81/02770
- US-A1- 2008 083 890

## Beschreibung

Die vorliegende Erfindung betrifft eine Vorrichtung zur Regulierung eines Volumenstroms in einem Schlauch, vorzugsweise einem Kunststoffschlauch, wie er beispielsweise in der Medizintechnik verwendet wird.

Vorrichtungen zur Regulierung des Volumenstroms in einem Schlauch sind im Stand der Technik bekannt. Derartige Vorrichtungen werden vorzugsweise in der Medizintechnik für die Verabreichung von Infusionen, Transfusionen und zur künstlichen Ernährung verwendet, wobei diese bei einer derartigen Verwendung als Infusions- oder Transfusions- oder Überleitungssysteme bzw. -bestecke bezeichnet werden. Diese Infusion- oder Transfusionsbestecke umfassen neben der Vorrichtung zur Regulierung des Volumenstroms weitere Bauteile, die insbesondere dazu dienen einen Infusionsbehälter mit einem intraarteriellen, intravenösen oder intraossären Zugang zu verbinden. So umfasst beispielsweise ein Infusionsbesteck einen Dorn zum Einstechen in einen Gummistopfen am Infusionsbehälter, eine Tropfkammer, einen Kunststoffschlauch, der beispielsweise als transparente Infusionsleitung ausgeführt sein kann, die Vorrichtung zum Regulieren des Volumenstroms und einen Anschlusskonnektor zum Verbinden des Bestecks mit einem Zugang, wie beispielsweise einem peripheren Venenkatheter.

Die Regulierung des Volumenstroms erfolgt gemäß den bekannten Vorrichtungen zur Regulierung eines Volumenstroms in einem Schlauch für den Einsatz in der Medizintechnik, vorzugsweise durch das Verändern des Schlauchquerschnittes durch ein Zusammenwirken eines Rollkörpers mit einer Innenfläche eines Gehäuses. In einem vollständig geöffneten Zustand der Vorrichtung wird ein maximaler Volumenstrom ermöglicht und in einer geschlossenen Position, bei welcher der Schlauchquerschnitt durch Quetschen vollständig reduziert ist tritt folglich kein Volumenstrom innerhalb des Schlauches auf.

Nachteilig in der im Stand der Technik bekannten Vorrichtung zur Regulierung eines Volumenstroms in einem Schlauch für den Einsatz in der Medizintechnik ist jedoch, dass unter anderem die Vorrichtung mit dem Infusions- oder Transfusionsbesteck vormontiert bereitgestellt werden muss, wobei ein gegebenenfalls notwendiges Abnehmen der Vorrichtung von dem entsprechenden Besteck nicht ermöglicht wird. Dies kann jedoch insbesondere dann notwendig sein, wenn das Infusions- oder Transfusionsbesteck nicht alleinstehend verwendet wird, sondern beispielsweise zusammen mit einer Infusionspumpe verwendet wird, mit welcher über ein entsprechendes Steuerungs- und Dosiersystem volumetrisch genaue Dosiermengen vorgegeben und sichergestellt werden können.

WO-A-81/02770 zeigt die Merkmale das Oberbegriffs des Anspruchs 1.

Aufgabe der vorliegenden Erfindung ist es die im Stand der Technik bekannten Nachteile bezüglich dem Einsatz und der Ausführung von Vorrichtungen zur Regulierung eines Volumenstrom in einem Schlauch für den Einsatz in der Medizintechnik wenigstens teilweise zu verbessern.

Die folgende Aufgabe wird durch eine erfindungsgemäße Klemme zur Regulierung eines Volumenstroms in einem Kunststoffschlauch gemäß Anspruch 1 gelöst. Bevorzugte, alternative Ausgestaltungsformen der Erfindung sind Gegenstand der Unteransprüche.

Erfindungsgemäß weist die Vorrichtung - beziehungsweise Klemme - zur Regulierung eines Volumenstroms in einem Kunststoffschlauch ein Gehäuse auf, in welchem neben wenigstens einem Kunststoffschlauch mit einem vorgegebenen Strömungsquerschnitt und ein, in dem Gehäuse längsverschieblich in einer Führung aufgenommenen Rads angeordnet sind. Dabei ist insbesondere das Rad, der Kunststoffschlauch und wenigstens eine Innenfläche des Gehäuses in der Art angeordnet, dass der Kunststoffschlauch in wenigstens einer ersten Position des Rades in Bezug auf das Gehäuse in seinem Strömungsquerschnitt maximal geöffnet ist und in wenigstens einer zweiten Position des Rades in Bezug auf das Gehäuse der Strömungsquerschnitt des Kunststoffschlauches wenigstens abschnittsweise fluiddicht geschlossen ist. Die erfindungsgemäße Vorrichtung ist dadurch gekennzeichnet, dass das Gehäuse vorzugsweise zweiteilig ausgeführt ist und insbesondere aus einem Deckel und einer Basis gebildet wird. Dabei sind der Deckel und die Basis derart ausgeführt, dass wenigstens eines dieser Bauteile im Querschnitt eine im Wesentlichen u-förmige Ausgestaltung aufweist und die beiden Bauteile vorzugsweise in Längsrichtung derart zueinander verschiebbar angeordnet sind, dass in der getrennten Position der beiden Bauteile der Innenraum des Gehäuses wenigstens zum Einlegen des Kunststoffschlauches freigegeben ist.

Gemäß einer weiteren, besonders bevorzugten Ausführungsform der erfindungsgemäßen Vorrichtung ist der Kunststoffschlauchs insbesondere ein flexibler, beziehungsweise hochflexibler Kunststoffschlauch, der vorzugweise transparent ausgeführt ist und gemäß einer weiteren, besonders bevorzugten Ausführungsform einen lichten Innendurchmesser aufweist, welcher vorzugsweise zwischen 0,2 mm und 10 mm liegt.

Gemäß einer weiteren, besonders bevorzugten Ausführungsform ist das Rad, welches längsverschieblich in einer Führung des Gehäuses aufgenommen ist mit einem vorgegebenen Umfangsquerschnitt ausgeführt und drehbar mittels zweier Achsabschnitte in einer Führung des Gehäuses gelagert.

Gemäß einer weiteren, besonders bevorzugten Ausführungsform ist hierbei die Führung für das Rad insbesondere als Rampe ausgebildet und weist vorzugsweise die Form einer Längsnut auf. Dabei ist die Längsnut vorzugsweise in dem Innenbereich des Gehäuses angeordnet, wobei sich die Längserstreckung des Gehäuses durch dessen Haupterstreckungsrichtung ergibt, entlang derer auch im Funktionsbetrieb im Wesentlichen der Kunststoffschlauch angeordnet ist.

Gemäß einer weiteren, besonders bevorzugten Ausführungsform ist die Führung, welche innerhalb des Gehäuses angeordnet ist, derart ausgestaltet, dass sie in Abhängigkeit des mittleren Raddurchmessers und der lichten Innenweite des Gehäuses das Rad in der ersten Position so weit von der Innenwandung des Gehäuses beabstandet, dass der Strömungsquerschnitt des Kunststoffschlauchs vollständig freigegeben ist. Wenigstens in einer zweiten Position des Rades wird ferner der Strömungsquerschnitt, insbesondere durch Quetschung des Kunststoffschlauchs, so zwischen der Innenwandung des Gehäuses und der Umfangskontur des Rades verschlossen, dass wenigstens in diesem Abschnitt ein Volumenstrom innerhalb des Kunststoffschlauches nicht auftritt und dieser somit fluiddicht verschlossen ist.

Gemäß einer weiteren, besonders bevorzugten Ausführungsform lässt sich der Strömungsquerschnitt des Schlauches zwischen der geöffneten und der verschlossenen Position des Rades in Bezug auf das Gehäuse schrittweise verändern, wobei gemäß einer weiteren, besonders bevorzugten Ausführungsform insbesondere die Bereiche, in denen ein vollständiges Schließen beziehungsweise ein vollständiges Freigeben des Strömungsquerschnittes möglich ist, nicht nur auf einzelne Punkte beschränkt sind, sondern in größeren Stellbereichen sichergestellt sind.

Gemäß einer weiteren, besonders bevorzugten Ausführungsform der vorliegenden Erfindung sind insbesondere im funktionsfähigen Zusammenbau des Gehäuses, der Deckel und die Basis des Gehäuses durch eine Führung, insbesondere eine Linearführung miteinander kraftschlüssig verbunden. Solch eine Führung kann insbesondere eine Flachführung, eine Prismenführung, eine Schwalbenschwanzführung oder eine Trapezführung sein, die insbesondere dazu dient, dass ein unbeabsichtigtes Trennen der beiden Bauteile im Betrieb verhindert wird.

Flachführungen haben dabei den Vorteil, dass sie einfach zu bearbeiten sind und eine hohe Steifigkeit besitzen. Damit die Führung einwandfrei funktionieren kann, muss eine so genannte Schmalführung verwendet werden. Diese zeichnet sich dadurch aus, das die seitliche Führung sehr schmal ausfallen muss, um wenigstens ein Verkanten vorzubeugen.

Prismenführungen sind von einer Dreiecksform abgeleitet und führen die Bauteile in zwei Richtungen, so dass zusätzliche Schmalführungen entfallen können. Die beste konstruktive Lösung dafür ist in der Prismen-Flachführung zu sehen, welche statisch bestimmt ist. Unter Wärmeeinwirkung können sich die Bauteile quer zur Bewegungsrichtung ausdehnen, ohne dass es zu Verspannungen kommen kann.

Die Schwalbenschwanzführung ist eine weitere Führungsart, die sich aus der Dreiecksform ableiten lässt. Diese Führungsart benötigt nur vier Führungsflächen und kann daher relativ klein gebaut werden. Durch die heutigen modernen Produktionsmaschinen und -prozesse stellen die erforderlichen Genauigkeiten kein Problem mehr dar.

Gemäß einer weiteren, besonders bevorzugten Ausführungsform kann die unbeabsichtigte Trennung der beiden Bauteile, das heißt ein versehentliches Öffnen des Deckels von der Basis des Gehäuses durch zusätzliche Sicherungsmedien vermieden werden. Hierzu kann insbesondere eine Rastung, ein Sicherungsstift, eine Lasche, eine Verjüngung der Führung im Endbereich zur Verklemmung des Deckels mit der Basis, eine insbesondere ein akustisches Signal gebende Schnappverbindung, Kombinationen hiervon und dergleichen verwendet werden.

Gemäß einer weiteren, besonders bevorzugten Ausführungsform kann ein Sicherungsmedium in der Art vorgesehen sein, dass ein einmal zusammengebautes Gehäuse anschließend nur noch durch Zerstörung des Gehäuses geöffnet werden kann. Eine weitere, alternative Ausführungsform bietet ein Sicherungsmedium, mit welchem insbesondere ein wiederholtes Schließen und Öffnen der beiden Bauteile gewährleistet wird, wobei insbesondere trotzdem berücksichtigt wird, dass ein versehentliches Öffnen beziehungsweise Verschieben des Deckels zur Basis vermieden wird.

Gemäß einer weiteren, besonders bevorzugten Ausführungsform der vorliegenden Erfindung ist insbesondere das Rad innerhalb der Führungsnut vorzugsweise in der Basis des Gehäuses vormontiert.

Gemäß einer weiteren, besonders bevorzugten Ausführungsform weist das Rad auf der Umfangskontur eine Rillung auf, welche vorzugsweise in einem vorgegebenen Winkel zur Rotationsachse ausgerichtet ist.

Gemäß einer weiteren, besonders bevorzugten Ausführungsform ist wenigstens das Gehäuse, einzelne Bauteile des Gehäuses oder das Sicherungsmedium aus einem Kunststoff hergestellt, der aus einer Gruppe ausgewählt ist, welche vorzugsweise Duro- und thermoplastische Kunststoffe, insbesondere Polyphenylensulfid, Polypropylen, Poly-1-buten, Polyvinylchlorid, Polyvinylidenchlorid, Polymethylmetaacrylat, Polyacrylnitril, Polystyrol, Polyacetal, Polyvinylalkohol, Polyvinylacetat, Ionomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Kombinationen hiervon und dergleichen aufweist.

Darüber hinaus ist wenigstens das Gehäuse, das Rad und/oder das Sicherungsmedium im Spritzgussverfahren hergestellt.

Gemäß einer weiteren besonders bevorzugten Ausführungsform der vorliegenden Erfindung ist insbesondere die Klemmkraft zur Quetschung des Kunststoffschlauches in der geschlossenen Position derart ausgeführt, dass diese einen Wert zwischen 10 N und 400 N aufweist und vorzugsweise eine Fluiddichtheit des Kunststoffschlauches bereitstellt, die auch bei einem anliegenden Fluiddruck im Kunststoffschlauch zwischen 0,1 bar und 8 bar aufrechterhalten wird.

Ein weiterer besonderer Vorteil der vorliegenden Erfindung ergibt sich in der Verwendung der Klemme bei der Transfusion, Enteral- und Parenteralfusion und im Einsatz mit Sondennahrungssystemen, Pumpenüberleitsystemen, Schwerkraftinfusionen und allgemeinen Überleitungssystemen. Darüber hinaus ergibt sich ein weiterer Vorteil dadurch, dass insbesondere bei Verwendung von integrierten Klemmsystemen oder dem Einsatz von Pumpen durch die nachträgliche Montage der erfindungsgemäßen Klemme eine Vorrichtung bereit gestellt wird, mit welcher sich auch nachträglich Einfluss auf den Volumenstrom in einem Schlauch nehmen lässt, wenn beispielsweise die Pumpe versagt oder die integrierte Klemme defekt ist.

Nachfolgend wird die Erfindung anhand verschiedener Ausführungsbeispiele gezeigt, wobei ausdrücklich darauf hingewiesen wird, dass die vorliegende Erfindung durch die nachfolgend ausgeführten Ausführungsbeispiele nicht beschränkt wird, sondern dass diese lediglich mögliche Ausführungsbeispiele wiedergibt, welchen der Fachmann entnehmen kann, dass darüber hinausgehende Änderungen und Abwandlungen auch im Sinn der vorliegenden Erfindung liegen.

Dabei zeigen:
Figur 1 eine perspektivische Seitenansicht der zusammengebauten Vorrichtung zur Regulierung eines Volumenstroms in einem medizinischen Schlauch,
Figur 2 eine perspektivische Explosionsdarstellung der Vorrichtung zur Regulierung eines Volumenstroms,
Figur 3 einen Längsschnitt durch die Vorrichtung zur Regulierung eines Volumenstroms,
Figur 4 eine perspeketivische Seitenansicht der Vorrichtung zur Regulierung eines Volumenstroms ohne den Schlauch,
Figur 5 eine Draufsicht auf die Vorrichtung zur Regulierung eines Volumenstroms in einem Schlauch entlang aus Figur 3,
Figuren 6 - 8 Seitenansichten der Vorrichtung gemäß Fig. 4.

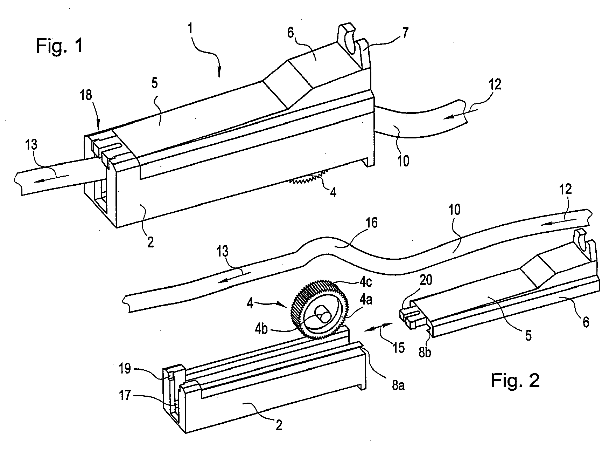

Figur 1 zeigt eine perspektivische Seitenansicht einer montierten Vorrichtung zur Regulierung eines Volumenstroms, bei der bereits der Schlauch 10 in dem Gehäuse 1 mit dem Deckel 5, der Basis 2 und das Rad 4 im funktionsfähigen Zustand zusammengesetzt sind. Dabei zeigt die Darstellung neben den einzelnen Bauteilen auch Details der Ausgestaltung.

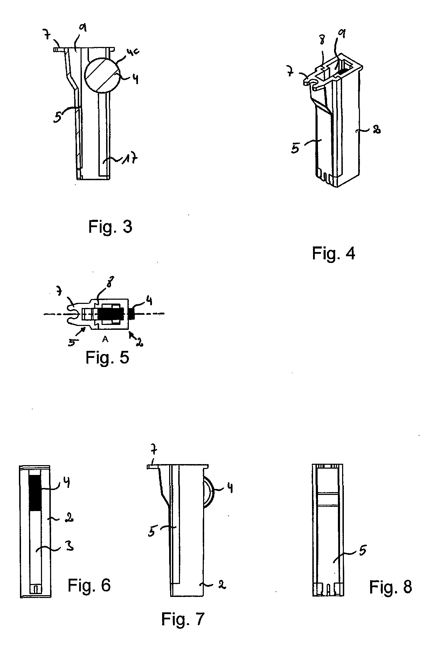

Die Figuren 6 - 8 zeigen Seitenansichten der Ausführungsform gemäß Fig. 3 zur Verdeutlichung einer möglichen Ausgestaltungsform oder erfindungsgemäßen Vorrichtung.

In Bezug auf das Gehäuse 1 ist die Basis 2 mit einer Längsausnehmung 3 zu erkennen, durch welche über den Verschiebeweg der Zugriff für die Verstellung durch den Bediener gegeben ist.

In dem rechten oberen Bereich ist die Erhöhung für die "Offenposition" der Vorrichtung zu erkennen, welche nach links hin durch das Heranführen der Innenseite des Gehäuses auf die Außenkontur des Rades 4 zu der gewünschten Quetschung des Schlauches 10 mit einem fluiddichtem Verschluss des Schlauches 10 führt.

Mit dem Bezugszeichen 7 ist eine Fixiereinrichtung für den Schlauch 10 dargestellt, in welcher insbesondere bei einer fertig vorbereiteten Infusion der Anschlussbereich des Schlauches mit dem Zugang für beispielweise einen peripheren Venenkatheter befestigt werden kann, um ein Herunterhängen und ggf. Verschmutzen des entsprechenden Bereiches zu vermeiden. Mit den Bezugszeichen 12 und 13 ist die bevorzugte Flussrichtung des Fluids innerhalb des Kunststoffschlauches 10 angedeutet. Mit dem Bezugzeichen 18 ist ein Sicherungsmedium gekennzeichnet, durch welches ein unbeabsichtigtes Versetzens des Deckels 5 von der Basis 2 vermieden wird.

Figur 2 ist eine perspektivische Explosionsdarstellung der Ausführungsform nach Fig. 1, wobei auch hier neben dem Schlauch 10 das Gehäuse 1 mit seiner Basis 2 der Deckel 5 und das Rad 4 zu erkennen sind. Mit der Darstellung wird ferner deutlich, wie gemäß der hier gezeigten Ausführungsform insbesondere die Verbindung zwischen dem Deckel 5 und der Basis 2 durch eine Schwalbenschwanzführung 8a und 8b bewirkt wird. Der Eingriff der Schwalbenschwanzfeder 8a in die Schwalbenschwanznut 8b wird durch Verschieben entlang dem Wechselpfeil 15 bewirkt. Das Rad 4 mit seiner Basis 4a und der Rotationsachse 4b zeigt auf seiner Umfangsfläche eine Riffelung 4c, die sowohl eine verbesserte Bedienbarkeit, als auch eine mehr oder weniger starke lineare Druckkraft auf den Schlauch 10 insbesondere in der geschlossenen Position bereit stellt. Die Drehachse 4b ist in der zusammengebauten Version in der Nut 17 der Basis 2 aufgenommen. Der in der Figur 2 dargestellte Schlauch 10 entspricht in seiner Darstellung dem Verlauf in einer Funktionsposition, wobei mit dem Abschnitt 16 gezeigt wird, dass sich der Schlauch 10 im Wesentlichen dem Außenradius des Rades 4 anpasst. Ferner ist in der Figur 2 der Aufbau als Sicherungsmedium zu erkennen, welche gemäß der hier gezeigten Ausführungsform aus einem zweiseitigen Hacken 20 im Deckel 5 und Rastausnehmungen 19 in der Basis 2 gebildet wird.

Figur 3 ist ein Längsschnitt durch die Vorrichtung aus Figur 1. In dieser Zeichnung ist klar zu erkennen, dass oben die geöffnete Position, das heißt der Bereich ist, in welchem der Strömungsquerschnitt des Kunststoffschlauches 10 im Wesentlichen nicht durch das Rad 4 verändert wird und somit vollständig geöffnet ist, bereitgestellt wird, wohingegen im unteren Bereich der Schlauch aufgrund der Quetschung zwischen der Außenkontur 4c des Rades 4 und der Innenkontur des Deckels 5 verschlossen wird. Dabei ist das Rad 4 über seine Rotationsachse 4b in der Nut 17 aufgenommen, welche die Führung entlang eines vorgegebenen Verschiebeweges bestimmt.

Gemäß der hier dargestellten Ausführungsform wird auch deutlich, dass sich die Innenkontur des Deckels 5 im Wesentlichen über die Längserstreckung von oben nach unten in Bezug auf die Nut 17 annähert, so dass die gewünschte, nahezu kontinuierliche Reduzierung des Strömungsquerschnittes des Kunststoffschlauches 10 mit der Bewegung des Rads von oben nach unten bereitgestellt wird.

Figur 4 ist perspektivische Darstellung der Vorrichtung gemäß Figur 3 zur Verdeutlichung der Außenkontur des Gehäuses.

Figur 5 ist eine Draufsicht auf die Vorrichtung gemäß Figur 3, wobei dieser Darstellung zu entnehmen ist, wie das Rad 4 in Bezug auf die Innenkontur in der Offenposition angeordnet ist. Ferner ist die Schwalbenschwanzführung 8 zu erkennen.

Figuren 6 bis 8 sind Seitenansichten der Figur 4 zur Verdeutlichung der Außenstruktur der hier gezeigten Ausführungsform.

## Patentansprüche

1. Klemme zur Regulierung eines Volumenstroms in einem Kunststoffschlauch (10) mit einem Gehäuse (1) zur Aufnahme des Kunststoffschlauchs (10) mit einem vorgegebenen Strömungsquerschnitt und einem in dem Gehäuse (1) längsverschieblich in einer Führung aufgenommenen Rad (4), wobei der Kunststoffschlauch (10) zwischen der Umfangskontur (4c) des Rades (4) und wenigstens einem Abschnitt einer Innenfläche des Gehäuses in der Art angeordnet ist, dass in wenigstens einer ersten Position des Rades (4) in Bezug auf das Gehäuse (1) der Strömungsquerschnitt des Kunststoffschlauchs (10) maximal geöffnet ist und in wenigstens einer zweiten Position des Rades (4) in Bezug auf das Gehäuse (1) der Strömungsquerschnitt abschnittsweise fluiddicht geschlossen ist, wobei
das Gehäuse (1) aus einem Deckel (5) und einer Basis (2) gebildet wird und wobei wenigstens der Deckel (5) und/oder die Basis (2) im Querschnitt u-förmig ausgebildet ist, **dadurch gekennzeichnet, dass** der Deckel (5) und die Basis (2) zueinander derart verschiebbar angeordnet sind,
dass in der getrennten Position der Innenraum des Gehäuses (1) wenigstens zum Einlegen des Kunststoffschlauchs (10) freigegeben ist.

2. Klemme gemäß Anspruch 1, **dadurch gekennzeichnet, dass**
der Kunststoffschlauch (10) insbesondere ein flexibler Kunststoffschlauch ist, der einen lichten Innendurchmesser aufweist, welcher zwischen 0,2 mm und 10 mm liegt.

3. Klemme gemäß einem der vorstehenden Ansprüche, **dadurch gekennzeichnet, dass**
das Rad (4) mit einem vorgegebenen Umfangsquerschnitt drehbar mittels zweier Achsabschnitte (4b) in der Führung (17) des Gehäuses (1) gelagert ist.

4. Klemme gemäß Anspruch 3, **dadurch gekennzeichnet, dass**
die Führung (17) als Rampe innerhalb des Gehäuses (1) ausgebildet ist und vorzugsweise die Form einer Längsnut aufweist.

5. Klemme gemäß Anspruch 3 oder 4, **dadurch gekennzeichnet, dass**
die Führung (17) in Abhängigkeit des mittleren Raddurchmessers und der lichten Innenweite des Gehäuses (1) das Rad (4) in der ersten Position so weit von der relevanten Innenwandung des Gehäuses (1) beabstandet, dass der Strömungsquerschnitt des Kunststoffschlauchs vollständig frei gegeben ist und in wenigstens der zweiten Position den Strömungsquerschnitt durch Quetschung des Kunststoffschlauches (10) zwischen der Innenwandung des Gehäuses (1) und der Umfangskontur (4c) des Rades (4) fluiddicht verschlossen wird.

6. Klemme gemäß einem der Ansprüche 3 bis 5, **dadurch gekennzeichnet, dass**
sich der Strömungsquerschnitt des Kunststoffschlauchs (10) über die Position des Rades (4) von der geöffneten Position in die geschlossene Position schrittweise verändert.

7. Klemme gemäß wenigstens einem der vorstehenden Ansprüche, **dadurch gekennzeichnet, dass**
der Deckel (5) und die Basis (2) des Gehäuses (1) durch eine Führung (8), insbesondere durch eine in Längsrichtung angeordnete Flachführung, Prismenführung, Schwalbenschwanzführung oder Trapezführung miteinander verbunden sind.

8. Klemme gemäß wenigstens einem der vorstehenden Ansprüche, **dadurch gekennzeichnet, dass**
ein Sicherungsmedium (18) zwischen der Basis (2) und dem Deckel (5) des Gehäuses (1) vorgesehen ist, welches ein versehentliches Öffnen bzw. Verschieben des Deckels (5) oder der Basis (2) verhindert, wobei ein solches Sicherungsmedium insbesondere eine Rastung, einen Sicherungsstift, eine Lasche, eine Verjüngung der Nut im Endbereich zur Verklemmung des Deckels mit der Basis, eine insbesondere ein akkustisches Signal gebende Schnappverbindung, Kombinationen hiervon und/oder dergleichen umfasst.

9. Klemme gemäß wenigstens einem der vorstehenden Ansprüche, **dadurch gekennzeichnet, dass**
das Rad (4) vorzugsweise in der Basis (2) vormontiert ist.

10. Klemme gemäß wenigstens einem der vorstehenden Ansprüche, dadurch gekennzeichet, dass
das Rad (4) auf der Umfangskontur (4c) eine Rillung aufweist, welche in einem vorgegebenen Winkel zur Rotationsachse ausgerichtet ist.

11. Klemme gemäß wenigstens einem der vorstehenden Ansprüche, **dadurch gekennzeichnet, dass**
wenigstens das Gehäuse (1) aus einem Kunststoff hergestellt wird, der aus einer Gruppe ausgewählt ist, welche vorzugsweise duro- und thermoplastische Kunststoff und insbesondere Polyphenylensulfid, Polypropylen, Poly-1-buten, Polyvinylchlorid, Polyvinylidenchlorid, Polymethylmetaacrylat, Polyacrylnitril, Polystyrol, Polyacetal, Polyvinylalkohol, Polyvinylacetat, Ionomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Kombinationen hiervon und dergleichen umfasst.

12. Klemme gemäß wenigstens einem der vorstehenden Ansprüche, **dadurch gekennzeichnet, dass**
die Klemme, einzelne Bauteile hiervon, wie beispielsweise das Rad (4) und/oder das Gehäuse (1) im Spritzgussverfahren hergestellt wird.

13. Klemme gemäß wenigstens einem der vorstehenden Ansprüche, **dadurch gekennzeichnet, dass**
die Klemmkraft zur Quetschung des Kunststoffschlauchs (10) in der geschlossenen Position einen Wert zwischen 10 N und 400 N aufweist und vorzugsweise die Fluiddichtheit bei einem anliegenden Fluiddruck im Kunststoffschlauch (10) zwischen 0,1 bar und 8 bar gewährleistet.

## Claims

1. A clamp for regulating a volume flow in a plastic hose (10), said clamp comprising a housing (1), for receiving the plastic hose (10) with a predetermined flow cross section, and a wheel (4), which is received in a longitudinally displaceable manner in a guide in the housing (1), wherein the plastic hose (10) is arranged between the circumferential contour (4c) of the wheel (4) and at least one portion of an inner surface of the housing in such a way that, in at least a first position of the wheel (4) in relation to the housing (1), the flow cross section of the plastic hose (10) is opened to the maximum, and, in at least a relation second to the position housing of the wheel (1), the flow (4) in cross section is closed in a fluid-tight manner at least in some parts,
whereby
the housing (1) is formed by a cover (5) and a base (2), at least the cover (5) and/or the base (2) having a U-shaped cross section, the cover (5) and the base (2) being displaceable relative to each other in such a way that, in the separated position, the interior of the housing (1) is freed at least for the insertion of the plastic hose (10).

2. The clamp according to claim 1,
**characterized in that**
the plastic hose (10) is in particular a flexible plastic hose having a clear internal diameter of between 0.2 mm and 10 mm.

3. The clamp according to any of the preceding claims, **characterized in that**
the wheel (4) with a predetermined circumferential cross section is mounted rotatable in the guide (17) of the housing (1) by means of two shaft portions (4b).

4. The clamp according to claim 3, **characterized in that**
the guide (17) is designed as a ramp inside the housing (1) and preferably has the shape of a longitudinal groove.

5. The clamp according to claim 3 or 4, **characterized in that**
the guide (17) depending on the mean wheel diameter and on the clear inside width of the housing (1), spaces the wheel (4) of the first position so far apart from the relevant inner wall of the housing (1) that the flow cross section of the plastic hose is completely freed, and, in at least the second position, the flow cross section is closed in a fluid-tight manner by means of the plastic hose (10) being squeezed between the inner wall of the housing (1) and the circumferential contour (4c) of the wheel (4).

6. The clamp according to one of claims 3 through 5, **characterized in that**
the flow cross section of the plastic hose (10) changes steps, via the position of the wheel (4), from the opened position to the closed position.

7. The clamp according to at least one of the preceding claims, **characterized in that**
the cover (5) and the base (2) of the housing (1) are connected to each other by a guide (8), in particular by a longitudinally arranged flat guide, prismatic guide, dovetail guide or trapezoid guide.

8. The clamp according to at least one of the preceding claims, **characterized in that**
a safety means (18) is provided between the base (2) and the cover 5) of the housing (1) and prevents inadvertent opening or movement of the cover (5) or of the base (2), such a safety means comprising in particular a catch, a locking pin, a clip, a tapering of the groove in the end area for the purpose of clamping the cover to the base, a snap-fit connection emitting in particular an acoustic signal, combinations of these and/or similar.

9. The clamp according to at least one of the preceding claims, **characterized in that**
the wheel (4) is preferably pre-assembled in the base (2).

10. The clamp according to at least one of the preceding claims, **characterized in that**
the circumferential contour (4c) of the wheel (4) has a fluting that is oriented at a predetermined angle with respect to the rotation axis.

11. The clamp according to at least one of the preceding claims, **characterized in that**
at least the housing (1) is made of a plastic chosen from a group that preferably comprises thermosetting plastic and thermoplastic, and in particular polyphenylene sulfide, polypropylene, poly-1-butene, polyvinyl chloride, polyvinylidene chloride, polymethyl methacrylate, polyacrylonitrile, polystyrene, polyacetal, polyvinyl alcohol, polyvinyl acetate, ionomers, fluoroplastic, polyethylene, polyamide, in particular a partially aromatic polyamide, polycarbonate, polyester, polyphenylene oxide, polysulfone, polyvinyl acetal, polyurethane, and chlorinated polyether, cellulose nitrate, cellulose acetate, cellulose ether, phenolic resin, urea resin, thiourea resin, melamine resin, alkyl resin, allyl resin, silicone, polyimide, polybenzimidazole, epoxy resin, casein plastic, crosslinked polyurethane, unsaturated polyester resin, combinations of these and similar.

12. The clamp as claimed in at least one of the preceding claims, **characterized in that**
the clamp, individual components thereof, for example the wheel (4) and/ or the housing (1), is produced by injection molding.

13. The clamp as claimed in at least one of the preceding claims, **characterized in that**
the clamping force for squeezing the plastic hose (10) in the closed position has a value of between 10N and 400N and preferably ensures the fluid tightness at a fluid pressure in the plastic hose (10) of between 0.1 bar and 8 bar.

## Revendications

1. Pince destinée à la régulation d'un débit volumétrique dans un tuyau en plastique (10) avec une boîte (1) pour loger le tuyau en plastique (10) avec une section mouillée prescrite et une roue (4) logée de manière mobile longitudinale dans la boîte (1) dans une coulisse, le tuyau en plastique (10) étant disposé entre le contour périphérique (4c) de la roue (4), et au moins une section d'une surface intérieure de la boîte de manière à ce que, dans au moins une première position de la roue (4) par rapport à la boîte (1), la section mouillée du tuyau en plastique (10) est ouverte au maximum et dans au moins une deuxième position de la roue (4) par rapport à la boîte (1), la section mouillée est fermée par section de manière étanche aux liquides,
**caractérisée en ce que**
la boîte (1) est constituée d'un couvercle (5) et d'une base (2), et au moins le couvercle (5) et/ou la base (2) étant agencés en forme de u dans la section transversale et étant disposés l'un par rapport à l'autre de manière mobile, de sorte que, dans la position séparée, l'espace intérieur de la boîte (1) est libéré au moins pour la pose du tuyau en plastique (10).

2. Pince selon la revendication 1, **caractérisée en ce que**
le tuyau en plastique (10) est notamment un tuyau flexible en plastique, qui présente un diamètre intérieur de passage qui se trouve entre 0,2 mm et 10 mm.

3. Pince selon une quelconque des revendications précédentes, **caractérisée en ce que**
la roue (4) est logée avec une section transversale périphérique prescrite de manière rotative dans la coulisse (17) de la boîte (1) au moyen de deux sections d'axe (4b).

4. Pince selon la revendication 3, **caractérisée en ce que**
la coulisse (17) est constituée comme rampe à l'intérieur de la boîte (1) et présente de préférence la forme d'une rainure longitudinale.

5. Pince selon les revendications 3 ou 4, **caractérisée en ce que**
en fonction du diamètre central de roue et du diamètre intérieur de passage de la boîte (1), la coulisse (17) éloigne la roue (4) dans la première position aussi loin de la paroi intérieure de la boîte (1), **en ce que** la section mouillée du tuyau en plastique est intégralement libérée et dans au moins la deuxième position, la section mouillée est fermée de manière étanche aux liquides par écrasement du tuyau en plastique (10) entre la paroi intérieure de la boîte (1) et le contour périphérique (4c) de la roue (4).

6. Pince selon une quelconque des revendications 3 à 5, **caractérisée en ce que**
la section mouillée du tuyau en plastique (10) passe par étape par l'intermédiaire de la position de la roue (4) de la position ouverte dans la position fermée.

7. Pince selon au moins une quelconque des revendications précédentes, **caractérisée en ce que**
le couvercle (5) et la base (2) de la boîte (1) sont reliés mutuellement par une coulisse (8), notamment une coulisse plate, une coulisse en prisme, une coulisse en queue d'hirondelle ou une coulisse trapézoïdale.

8. Pince selon au moins une quelconque des revendications précédentes, **caractérisée en ce que**
un élément de sécurité (18) est prévu entre la base (2) et le couvercle (5) de la boîte (1), qui empêche une ouverture ou un déplacement fortuits du couvercle (5) ou de la base (2), un tel élément de sécurité comprenant notamment un cran, une goupille de sécurité, une attache, une conicité de la rainure dans la zone terminale pour le serrage du couvercle avec la base, un assemblage à encliquetage émettant notamment un signal acoustique, des combinaisons de ceux-ci et des produits similaires.

9. Pince selon au moins une quelconque des revendications précédentes, **caractérisée en ce que**
la roue (4) est prémontée de préférence dans la base (2).

10. Pince selon au moins une quelconque des revendications précédentes, **caractérisée en ce que**
la roue (4) présente une cannelure sur le contour périphérique (4c), qui est alignée dans un angle prescrit par rapport à l'axe de rotation.

11. Pince selon au moins une quelconque des revendications précédentes, **caractérisée en ce que**
au moins la boîte (1) est fabriquée en un plastique qui est sélectionné à partir d'un groupe qui comporte de préférence du duroplastique et du thermoplastique et notamment du polysulfure de phénylène, du polypropylène, du poly-1-butylène, du chlorure de polyvinyle, du chlorure de polyvinylidène, du polyméthacrylate de méthyle, du polyacrylonitrile, du polystyrène, du polyacétal, de l'alcool polyvinylique, du polyacétate de vinyle, des ionomères, du plastique fluoré, du polyéthylène, du polyamide, notamment un polyamide aromatique partiel, du polycarbonate, du polyester, du polyphénylène oxyde, du polysulfone, de l'acétal polyvinylique, du polyuréthane, et du polyéther chloré, du nitrate de cellulose, de l'acétate de cellulose, de l'éther de cellulose, de la résine phénolique, de la résine d'urée, de la résine thio-urée, de la résine de mélamine, de la résine d'alcoyle, de la résine allylique, de la silicone, du polyimide, du polybenzimidazole, de la résine époxy, du plastique à la caséine, du polyuréthane réticulé, de la résine polyester non saturée, des combinaisons de ceux-ci et des produits similaires.

12. Pince selon au moins une quelconque des revendications précédentes, **caractérisée en ce que**
la pince, des composants individuels de celle-ci, par exemple la roue (4) et/ou la boîte (1) sont fabriquées au procédé de moulage par injection.

13. Pince selon au moins une quelconque des revendications précédentes, **caractérisée en ce que**
pour écraser le tuyau en plastique (10) dans la position fermée, la force de serrage présente une valeur entre 10 N et 400 N et garantit de préférence l'étanchéité aux liquides en présence d'une pression de liquide en suspens dans le tuyau en plastique (10) entre 0,1 bar et 8 bars.
